# EUROPEAN PATENT SPECIFICATION

(11) **EP 1 437 151 B1**
(45) Date of publication and mention of the grant of the patent: **14.06.2006**
(21) Application number: 03000746.2
(22) Date of filing: 13.01.2003
(51) Int. Cl.: A61M 5/50, A61M 5/32

(54) **Automatically retractable safety syringe**
Sicherheitsspritze mit automatischer ausziehbarer Nadel
Seringue de sécurité à aiguille automatiquement rétractable

(43) Date of publication of application: 14.07.2004
(73) Proprietor: Medicalchain International CORP., Taipei, Taiwan 104 (TW)
(72) Inventor: Medicalchain International CORP., Taipei, Taiwan 104 (TW)
(74) Representative: Beck, Michael Rudolf

(56) References cited:
- EP-A- 1 166 809
- FR-A- 2 653 667
- GB-A- 2 243 552
- US-A- 5 605 544
- US-A- 6 096 005

## Description

The present invention relates to an automatically retractable safety syringe according to the preamble portion of claim 1. The syringe is designed to be operated by single hand and to have an automatically retractable needle assembly not allowing for reuse.

Nowadays, since syringes have been widely used to inject medicine all around the world, needle sticking related injuries occur quite often; the sequelae caused by this kind of accident should not be overlooked because more than twenty kinds of diseases are infected through blood transmission, for example, HIV-1 (AIDS), Hepatitis B and C; it requires a considerable society cost for handling the follow-ups after injury accidents. The prior art safety syringe is usually operated manually by both hands; although the manufacturing cost thereof is low, its application is inconvenient and not safe enough either.

An automatically retractable safety syringe according to the preamble portion of claim is known from EP 1 166 809 A1, which discloses a retractable needle and retractable piston rod. The retraction of the needle is caused by a spring and the retraction of the piston rod is achieved by breaking it into two parts and subsequently telescoping it The mechanism for initiating the retraction particularly of the needle comprises a needle holder that is fixed on an inner wall of the tubular barrel. After completion of the injections stroke and after collapse of the piston rod, blades provided on the piston rod cut off the connection between the needle holder and inner wall, thereby releasing the spring that retracts the needle into the tubular barrel.

Further, GB 2 243 552 A discloses a singlo-use syringe having a retractable needle only. The needle is fixed on a needle holder that is locked between an outer tubular syringe barrel and an inner barrel. The inner barrel is inserted and fixed in the outer barrel. A compressed spring is arranged between the outer barrel and the needle holder. Further, the syringe comprises a piston rod having a piston head. In order to retract the needle after use of the syringe, the piston head unlocks the needle holder at the end of the injection stroke. The bias of the spring causes the needle holder and needle to retract. Moreover, in some embodiments, the piston head disengages from the piston rod. By this, it is possible to remove the piston rod, which would otherwise resume a position after loading of the syringe and thus project far beyond the rear end of the outer tubular syringe barrel.

Another syringe having a retractable needle is disclosed in US 6,096,005 A. The needle has a small needle holder biased by a spring. The needle holder is locked in its position by claws. These claws may spread upon actuation by the piston to release the needle holder and spring, which are thus retracted into the piston rod. Accordingly, US 6,096,005 A does not require a breakable piston rod. Moreover 6,096,005 A teaches a cap fixed on a ridge of the tubular barrel in a conventional manner.

The invention intends to provide a safety syringe of the type allowing the needle and piston rod to retract into its tubular barrel and intends to improve the needle retraction mechanism with regard to size and simple and safe handling.

This technical problem is solved by a syringe according to claim 1. Further embodiments of the invention are set forth in the claims.

The syringe of the present invention is designed to be operated by single hand, to have an automatically retractable needle assembly not allowing for reuse; the said automatically retractable safety syringe comprises a needle assembly with a cannula, a needle hub and a rubber O-ring thereof, a needle cap, a retracting spring, a breakable plunger, a plunger rubber gasket and a medicine barrel; in accordance with the structure of the said automatically retractable safety syringe of the present invention, since the breakable plunger is assembled inside the medicine barrel, after being pushed all the way to the end, it is forced to break automatically into two parts for reducing its length so as to provide enough space for the retracting spring to drive the needle assembly moving along the sliding passages to accomplish the automatically retracting action.

To enable a further understanding of the structural features and the technical contents of the present invention, the brief description of the drawings below is followed by the detailed description of the preferred embodiment. In the drawings:
- Figure 1: is an exploded drawing of the present invention.

- Figure 2: is an enlarged drawing of a needle head of the present invention.
- Figure 3: is a lateral view and exploded drawing of the present invention.
- Figure 4: is a lateral view and cross-sectional drawing of the present invention.
- Figure 4A: is a partially enlarged drawing of the present invention.
- Figure 4B: is another partially enlarged drawing of the present invention.
- Figure 4C: is also another partially enlarged drawing of the present invention.
- Figure 5: is a drawing of the application of the present invention.
- Figure 6: is another drawing of the application of the present invention.
- Figure 7: is a drawing of the retracting action of the needle assembly of the present invention.
- Figure 8: is another drawing of the retracting action of the needle assembly of the present invention.
- Figure 9: is a drawing of the present invention in a destructive state.

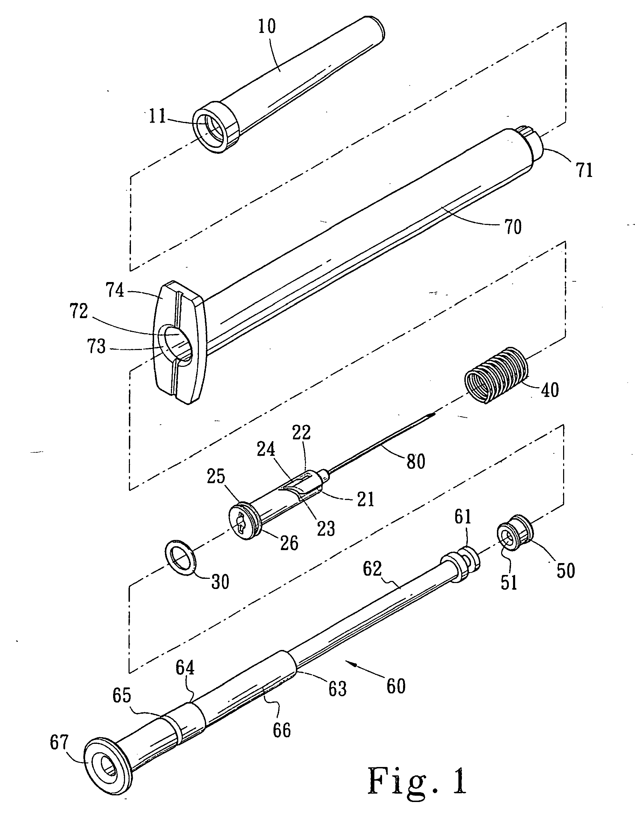

As indicated in FIGS. 1, the exploded drawings of the present invention, a needle assembly thereof has a cannula (80) and a needle hub (20) with one end thereof used for receiving the cannula (80) to fitly penetrate through its center; the circumference at the other end is disposed with an annular rubber gasket groove (25) and the convex torus thereof is disposed as a check plate (26) toward one end of an retracting spring (40); a plurality of concave positioning slots (22) are disposed in a proper area at two ends of the needle hub (20) to connect a plurality of proceeding slopes (24) tapered upwardly, a plurality of rotating slide passages (23) concaved inwardly and slanted for rotation as well as to connect a plurality of communicating slide passages (21) concaved inwardly to form an U-shaped slide passage for the needle assembly to make retracting action; a needle hub rubber gasket (30) is used for making a tight seal; an annular retracting spring (40) compresses and expands to retract the needle assembly; a plunger rubber gasket (50) is also used for making a tight seal; a breakable inwardly retracting plunger (60) is disposed with a long inwardly retracting rod (62) with one end disposed with a rubber clip head (61) to clip the plunger rubber gasket (50) and the other end thereof connected to one end of a hollow receiving rod (66) to form a breakable area (63) in an annular or annular block shape; the other end of the hollow receiving rod (66) is disposed with a push grip (67); an annular bottom check ring (64) is disposed in a proper area at the said two ends for controlling the bottom position of the syringe; an annular fastening annular groove (65) controls and fastens the breakable inwardly retracting plunger (60) to break into two parts and rotate with the needle assembly for completing inwardly retraction; the center of the hollow receiving rod (66) has a space for receiving the inwardly retracting rod (62); one end of a medicine barrel (70) is disposed with a plurality of slightly resilient retaining hook (71) pointing to the center for cooperating with the U-shape of the needle hub; the other end is disposed with a handle lug (74); the aperture of one end of a communicating inner barrel (72) is smaller than that of the inner barrel (72) to form a check ring for stopping the other end of the retracting spring (40); the other end of the said inner barrel (72) is disposed with an annular convex ring (73) for controlling and positioning the bottom check ring (64) and the fastening annular groove (65); the said inner barrel (72) has the space for receiving the needle assembly, the retracting spring (40), the breakable inwardly retracting plunger (60) and the plunger rubber gasket (50) thereof; a needle cap (10) covers to guard the needle assembly and fitly clips unto the retaining hook (71) of the medicine barrel (70); when the needle cap (10) is not separated from the retaining hook (71) of the medicine barrel (70), the retaining hook (71) does not eject for the needle assembly to retract.

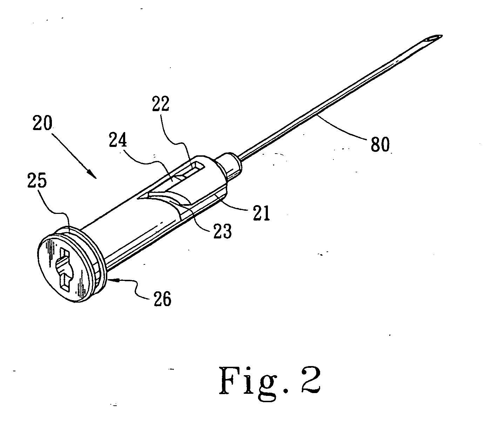

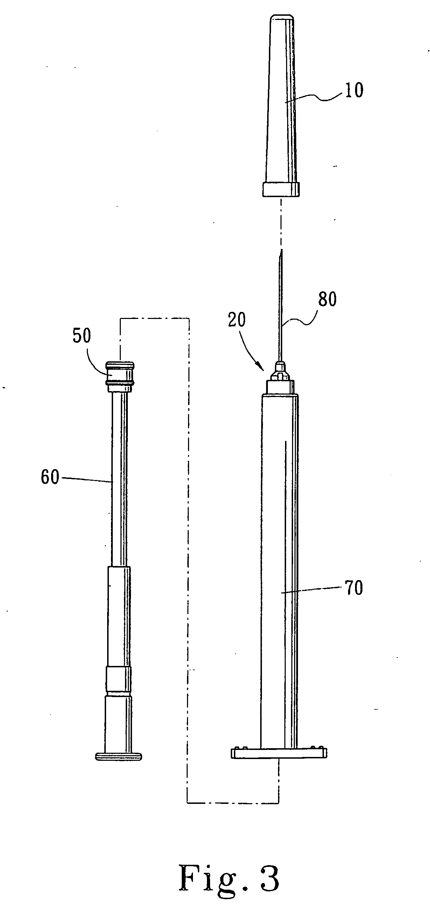

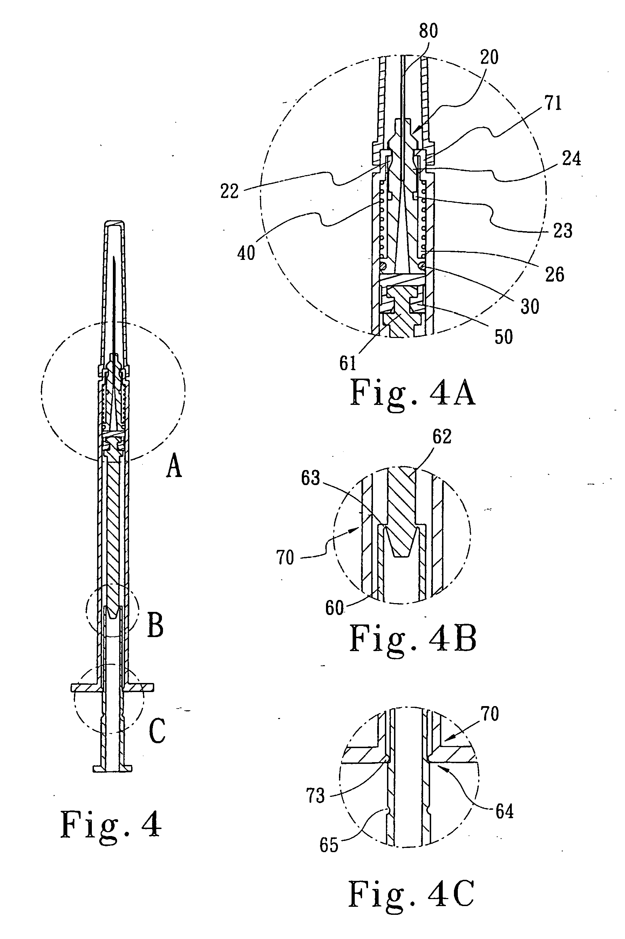

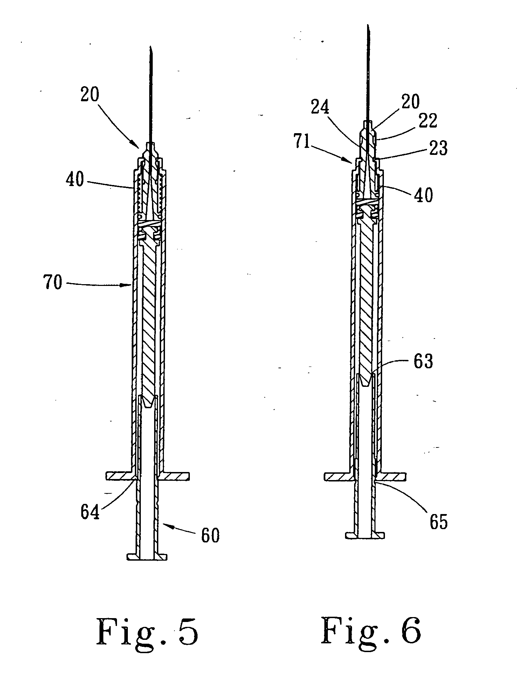

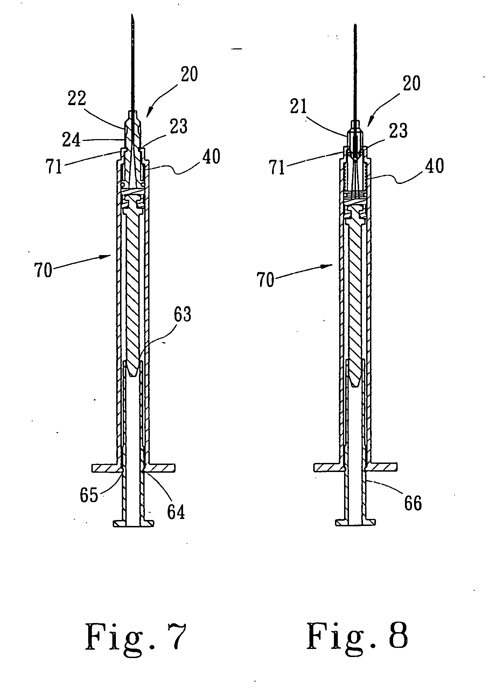

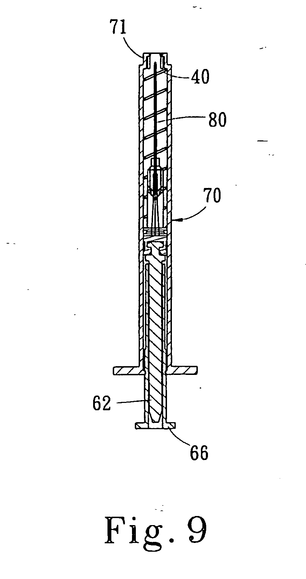

As indicated in FIGS. 2 to 4C, the retaining hook (71) of the medicine barrel (70) of the present invention retains into the positioning slot (22) of the needle hub (20) to prevent the needle assembly from retracting inwardly due to the tension of the spring (40), as shown in FIG. 4A; one end of the long inwardly retracting rod (62) connects to one end of the hollow receiving rod (66) to form the breakable area (63) in an annular or annular block shape, as shown in FIG. 4B; the annular convex ring (73) controls and positions the bottom check ring (64) and the fastening annular groove (65), as shown in FIG. 4C; referring to FIGS. 5 and 6, the retracting spring (40) has a compressed space; the needle hub (20) is positioned and the convex ring (73) is clipped at the bottom check ring (64); the breakable inwardly retracting plunger (60) is forced inwardly; the retaining hook (71) moves along the proceeding slope (24) to reach the rotating slide passage (23); the retracting spring (40) reaches the limit; the breakable inwardly retracting plunger (60) breaks into two parts; the fastening annular groove (65) fitly clips to the convex ring (73); as shown in FIGS. 7 and 8, after the breakable inwardly retracting plunger (60) breaks into two parts, the retracting spring (40) starts to expand to drive the needle hub (20) to rotate and detach completely from the retaining hook (71) along the communicating slide passage (21) till the needle assembly retracts all the way into the inner barrel (72); FIG. 9 indicates the completely destructive state of the plunger (60) of the present invention.

To assemble the present invention of an automatically retractable safety syringe, the retracting spring (40) is first placed through the convex ring (73) of the inner barrel (72), then one end of the needle assembly penetrates through the retracting spring (40); the check plate (26) on the other end stops one end of the retracting spring (40); the positioning slot (22) of the needle hub (20) is positioned and clipped to the retaining hook (71); the area of the retracting spring (40) is in a halfway compressed state; then the breakable inwardly retracting plunger (60) and the plunger rubber gasket (50) retain with a retaining hole (51) via the rubber clip head (61); finally, two sets are assembled and the needle cap (10) is covered onto the retaining hook (71) to finish the assembly.

In accordance with the structure of the said automatically retractable safety syringe of the present invention, after the breakable inwardly retracting plunger assembled inside the medicine barrel is pushed all the way to the end, it is forced to break automatically into two parts for reducing it's length so as to provide enough space for the retracting spring to drive the needle assembly moving along the sliding passages for accomplishing the action of automatic retraction; therefore the present invention has less parts and thereby requires lower manufacturing cost; furthermore, the application is simple and safe; the present invention can be manufactured as an automatically retractable safety syringe in a smaller volume.

It is of course to be understood that the embodiment described herein is merely illustrative of the principles of the invention and that a wide variety of modifications thereto may be effected by persons skilled in the art without departing from the scope of the invention as set forth in the claims.

## Claims

1. An automatically retractable safety syringe to be adopted for injecting medicine, comprising:
- a medicine barrel (70) having an internal body (72);
- a needle assembly having a needle hub (20), a cannula (80) and a needle hub rubber gasket (30) for making a tight seal;
- a retracting spring (40) co-operating with the needle assembly;
- a breakable inwardly retracting plunger (60) capable of breaking into two parts and a plunger rubber gasket (50) on the plunger for making a tight seal;
- a needle cap (10) for covering and guarding the needle assembly;
- wherein the internal body (72) of the medicine barrel (70) has the space for action of the needle assembly, the retracting spring (40), the breakable inwardly retracting plunger (60) and the plunger rubber gasket; the retracting spring (40) is adapted to store kinetic energy and to expand to receive the needle assembly in the interior space;
**characterized in that**
- the needle hub (20) is adapted to move and rotate in conjunction with preset sliding passage (22, 23, 24) for accomplishing an automatic retracting action;
- a retaining hook (71) is provided on the medicine barrel (70) to engage with said sliding passage (22, 23, 24);
- the needle cap (10) is adapeted to clip onto the retaining hook (71) so that when the needle cap (10) is not separated from the retaining hook (71), the retaining hook (71) is not able to eject and the needle assembly cannot retract either;
- wherein the breakable inwardly retracting plunger (60) is adapted to drive the needle assembly and further compress the retracting spring (40) from a partly compressed state; and
- wherein after the breakable inwardly retracting plunger (60) has been broken into two parts, the retracting spring (40) starts to expand to drive the needle assembly to rotate along the preset sliding passage (22, 23, 24) for accomplishing the automatically retracting action.

2. An automatically retracting safety syringe according to claim 1, **characterized in that** one end of the needle hub (20) receives the needle cannula (80) fitly penetrating the center thereof for flowing the medicine; the circumference of the other end is disposed with an annular rubber gasket groove (25); the said convex torus is disposed as a check plate (26) at one end of an retracting spring (40); a plurality of concave positioning slots (22) are disposed in a proper area at two ends of the needle hub (20) to connect a plurality of proceeding slopes (24) tapered upwards, a plurality of rotating slide passages (23) concaved inwardly and slanted for rotation as well as to connect a plurality of communicating slide passages (21) concaved inwardly to form at least more than two U-shaped slide passages for the needle assembly to make retracting action;

3. An automatically retracting safety syringe according to claim 1 or 2, **characterized in that** a breakable inwardly retracting plunger (60) is disposed with a long inwardly retracting rod (62) with one end thereof disposed with a rubber clip head (61) to clip with the plunger rubber gasket (50) and the other end thereof connected to one end of a hollow receiving rod (66) to form a breakable cutting edge area in an annular or annular block shape; the other end of the hollow receiving rod (66) is disposed with a push grip (67); an annular bottom check ring (64) is disposed in a proper area at the said two ends for controlling the bottom position of the syringe; an annular fastening annular groove (65) controls and fastens the breakable inwardly retracting plunger (60) to break into two parts and rotate with the needle assembly for completing inwardly retraction; the center of the circumference of the hollow receiving rod (66) has a space for receiving the inwardly retracting rod (62).

4. An automatically retracting safety syringe according to claim 1, 2 or 3, **characterized in that** one end of the cylindrical medicine barrel (70) is disposed with a plurality of slightly resilient retaining hooks (71) pointing to the center of the circumference for cooperating with the U-shape of the needle hub (20); the other end is disposed with a handle lug (74); the aperture of one end of a communicating inner barrel (72) is smaller than that of the inner barrel (72) to form a check ring for stopping the other end of the retracting spring (40); the other end of the said inner barrel (72) is disposed with an annular convex ring (73) for controlling and positioning the bottom check ring (64) and the fastening annular groove (65); the said inner barrel (72) has the space for receiving the needle assembly, the retracting spring (40), the breakable inwardly retracting plunger (60) and the plunger rubber gasket (50) thereof.

## Patentansprüche

1. Automatisch zurückziehbare Sicherheitsspritze zum Injizieren von Medikamenten, umfassend:
- einen Medikamentenzylinder (70) mit einem Innenkörper (72);
- eine Kanülenanordnung mit einem Kanülenhalter (20), einer Kanüle (80) und einer Gummidichtung (30) für den Kanülenhalter zur Bereitstellung einer engen Dichtung;
- eine Rückstellfeder (40), die mit der Kanülenanordnung zusammenwirkt;
- einen zerbrechbaren, einwärts zurückziehbaren Kolben (60), der in zwei Teile zerbrechbar ist, sowie eine Kolbengummidichtung (50) an dem Kolben zur Bereitstellung einer engen Dichtung;
- eine Kanülenkappe (10) zur Abdeckung und zum Schutz der Kanülenanordnung;
- wobei der Innenkörper (72) des Medikamentenzylinders (70) einen Raum für die Bewegung der Kanülenanordnung, der Rückstellfeder (40), des zerbrechbaren, einwärts zurückziehbaren Kolbens (60) und der Kolbengummidichtung aufweist; wobei die Rückstellfeder (40) geeignet ist, kinetische Energie zu speichern und sich auszudehnen, um die Kanülenanordnung in dem Innenraum aufzunehmen;
**dadurch gekennzeichnet, dass**
- der Kanülenhalter (20) geeignet ist, sich in Abhängigkeit einer vorgegebenen Gleitführung (22, 23, 24) zu bewegen und zu drehen, zur Bewerkstelligung einer automatischen Rückzugsbewegung;
- ein Rückhaltehaken (71) an dem Medikamentenzylinder (70) zum Eingriff mit der Gleitführung (22, 23, 24) vorgesehen ist;
- die Kanülenkappe (10) geeignet ist, auf dem Rückhaltehaken (71) zu verrasten, so dass, wenn die Kanülenkappe (10) nicht von dem Rückhaltehaken getrennt ist (71), der Rückhaltehaken (71) nicht in der Lage ist, auszulassen, und die Kanülenanordnung nicht zurückgezogen werden kann;
- wobei der zerbrechbare, einwärts zurückziehbare Kolben (60) geeignet ist, die Kanülenanordnung zu beaufschlagen und die Rückstellfeder (40) aus einem teilweise zusammengedrückten Zustand weiter zusammenzudrücken; und
- wobei, nachdem der zerbrechbare, einwärts zurückziehbare Kolben (60) in zwei Teile gebrochen ist, die Rückstellfeder (40) sich ausdehnt, um die Kanülenanordnung zu beaufschlagen, um entlang der Gleitführung (22, 23, 24) zu drehen, zur Bewerkstelligung der automatischen Rückzugsbewegung.

2. Automatisch zurückziehbare Sicherheitsspritze nach Anspruch 1, **dadurch gekennzeichnet, dass** ein Ende des Kanülenhalters (20) die Kanüle (80) aufnimmt, die in diesen eingepasst ist und sich durch dessen Mitte erstreckt, um ein Medikament hindurchzuleiten; der Umfang des anderen Endes mit einer ringförmigen Gummidichtungsnut (25) versehen ist; der konvexe Ring als Anschlagplatte (26) an einem Ende der Rückstellfeder (40) angeordnet ist; eine Vielzahl von ausgenommenen Positionierungsnuten (22) in einem geeigneten Bereich an zwei Enden des Kanülenhalters (20) angeordnet ist, um an eine Vielzahl von fortführenden, nach oben ansteigenden Neigungsabschnitten (24) anzuschließen, eine Vielzahl von ausgenommenen und zur Drehung angewinkelten Drehgleitführungen (23), die an eine Vielzahl von ausgenommenen Verbindungsgleitführungen (21) anschließen, um zumindest mehr als zwei U-förmige Gleitführungen für die Kanülenanordnung bereitzustellen, um die Rückzugsbewegung zu bewerkstelligen.

3. Automatisch zurückziehbare Sicherheitsspritze nach Anspruch 1 oder 2, **dadurch gekennzeichnet, dass** der zerbrechbare, einwärts zurückziehbare Kolben (60) mit einer langen einwärts zurückziehbaren Stange (62) ausgebildet ist, die an einem Ende mit einem Gummiklipskopf (61) versehen ist, um mit der Kolbengummidichtung (50) zu verrasten, und deren anderes Ende unter Ausbildung eines ringförmigen Bruchkantenbereichs mit einem Ende einer hohlen Aufnahmestange (66) verbunden ist; das andere Ende der hohlen Aufnahmestange (66) mit einem Druckgriff (67) versehen ist; ein Anschlagring (64) in einem geeigneten Bereich des zweiten Endes vorgesehen ist, um die Ausgangsstellung der Spritze einzustellen; eine Ringnut (65) den zerbrechbaren, einwärts zurückziehbaren Kolben (60) steuert und festlegt, um diesen in zwei Teile zu brechen und mit der Kanülenanordnung zu drehen, um die einwärts gerichtete Rückzugsbewegung abzuschließen; um die Mitte des Umfangs der hohlen Aufnahmestange (66) ein Raum zur Aufnahme der einwärts zurückziehbaren Stange (62) ausgebildet ist.

4. Automatisch zurückziehbare Sicherheitsspritze nach Anspruch 1, 2 oder 3, **dadurch gekennzeichnet, dass** ein Ende des zylindrischen Medikamentenzylinders (70) mit einer Vielzahl von geringfügig nachgiebigen Rückhaltehaken (71) versehen ist, die zu der Mitte des Umfangs weisen, zur Zusammenwirkung mit der U-Form des Kanülenhalters (20); das andere Ende mit einem Griffstück (74) versehen ist; die Öffnung an einem Ende des zugehörigen Innenzylinders (72) kleiner ist, als die des Innenzylinders (72), um einen Anschlagring zur Anlage für das andere Ende der Rückstellfeder (40) auszubilden; das andere Ende des Innenzylinders (72) mit einem vorspringenden Ring (73) versehen ist, zur Steuerung und Positionierung des Anschlagrings (64) für die Ausgangsstellung und der Ringnut (65) für die Festlegungsstellung; der Innenzylinder (72) einen Raum zur Aufnahme der Kanülenanordnung, der Rückstellfeder (40), des zerbrechbaren, einwärts zurückziehbaren Kolbens (60) und der Kolbengummidichtung (50) aufweist.

## Revendications

1. Seringue de sécurité rétractable automatiquement à adopter pour injecter des médicaments comprenant:
- un cylindre de médicament (70) ayant un corps interne (72);
- un montage d'aiguille ayant un moyeu d'aiguille (20), une cannule (80) et un joint d'étanchéité en caoutchouc de moyeu d'aiguille (30) pour faire un scellement étanche;
- un ressort de rappel (40) qui coopère avec le montage d'aiguille;
- un piston apte à se casser escamotable vers l'intérieur (60) capable de se casser en deux parties et un joint d'étancheité en caoutchouc de piston (50) sur le piston pour faire un scellement étanche;
- un capuchon d'aiguille (10) pour couvrir et protéger le montage d'aiguille;
- dans laquelle le corps intérieur (72) du cylindre de médicament (70) a l'espace d'action du montage d'aiguille, le ressort de rappel (40), le piston apte à se casser escamotable vers l'intérieur (60) et le joint d'étanchéité en caoutchouc du piston, le ressort de rappel (40) est adapté pour accumuler de l'énergie cinétique et pour se détendre pour recevoir le montage d'aiguille dans l'espace intérieur;
**caractérisée en ce**
- **que** le moyeu d'aiguille (20) est adapté pour se déplacer et tourner en relation avec un passage de glissement préréglé (22, 23, 24) pour accomplir une action de rappel automatique;
- un crochet de retenue (71) est prévu sur le cylindre de médicament (70) pour s'engrener avec ledit passage de glissement (22, 23, 24);
- le capuchon d'aiguille (10) est adapté pour se clipser sur le crochet de retenue (71) si bien que, lorsque le capuchon d'aiguille (10) n'est pas séparé du crochet de retenue (71), le crochet de retenue (71) n'est pas capable de s'éjecter et le montage d'aiguille ne peut pas non plus se rétracter;
- dans laquelle le piston escamotable vers l'intérieur capable de se casser (60) est adapté pour entraîner le montage d'aiguille et continuer à comprimer le ressort de rappel (40) à partir d'un état partiellement comprimé; et
- dans laquelle, après que le piston escamotable vers l'intérieur capable de se casser (60) s'est cassé en deux parties, le ressort de rappel (40) commence à se détendre pour entraîner le montage d'aiguille pour tourner le long du passage de glissement préréglé (22, 23, 24) pour accomplir l'action de rappel automatique.

2. Seringue de sécurité rétractable automatiquement selon la revendication 1, **caractérisée en ce qu'**une extrémité du moyeu d'aiguille (20) reçoit la canule d'aiguille (80) pénétrant de manière ajustée au centre de celui-ci pour faire s'écouler le médicament, la circonférence de l'autre extrémité est disposée avec un joint d'étanchéité annulaire en caoutchouc (25), ledit tore convexe est placé comme une plaque d'arrêt (26) à une extrémité d'un resort de rappel (40), une pluralité de fentes de positionnement concaves (22) sont placées dans une zone appropriée aux deux extrémités du moyeu d'aiguille (20) pour relier une pluralité de pentes d'avancement (24) effilées vers le haut, une pluralité de passages coulissants rotatifs (23) étant concaves vers l'intérieur et inclinés pour rotation ainsi que pour relier une pluralité de passages coulissants communicants (21) concaves vers l'intérieur pour former au moins plus de deux passages coulissants en forme d'U pour le montage d'aiguille pour faire l'action de rétraction.

3. Seringue de sécurité rétractable automatiquement selon la revendication 1 ou 2, **caractérisée en ce qu'**un piston escamotable vers l'intérieur capable de se casser (60) est placé avec une longue tige rétractable vers l'intérieur (62) avec une extrémité placée avec une tête de clipsage en caoutchouc (61) pour se clipser avec le joint d'étanchéité en caoutchouc (50) de piston et l'autre extrémité de celui-ci reliée à une extrémité de la tige creuse de logement (66) pour former une zone d'arête de coupe capable de se casser dans une forme annulaire ou de bloc annulaire, l'autre extrémité de la tige creuse de logement (66) est placée avec une prise de poussée (67), un anneau annulaire de contrôle du fond (64) est placé dans une zone appropriée aux deux extrémités pour contrôler la position de fond de la seringue, une rainure annulaire de fixation annulaire (65) contrôle et fixe le piston escamotable vers l'intérieur capable de se casser (60) pour se casser en deux parties et tourner avec le montage d'aiguille pour effectuer un retrait vers l'intérieur, le centre de la circonférence de la tige creuse de logement (66) a un espace pour loger la tige escamotable vers l'intérieur (62).

4. Seringue de sécurité rétractable automatiquement selon la revendication 1, 2 ou 3, **caractérisée en ce qu'**une extrémité du cylindre de médicament cylindrique (70) est placée avec une pluralité de crochets de retenue légèrement élastiques (71) orientés vers le centre de la circonférence pour coopérer avec la forme en U du moyeu d'aiguille (20, l'autre extrémité étant placée avec un oeillet de manipulation (74), l'ouverture d'une extrémité du cylindre intérieur communiquant (72) étant plus petite que celle du cylindre intérieur (72) pour former un anneau d'arrêt pour arrêter l'autre extrémité du ressort de rappel (40), l'autre extrémité dudit cylindre intérieur (72) étant disposée avec un anneau convexe annulaire (73) pour contrôler et positionner l'anneau de contrôle du fond (64) et la rainure annulaire de fixation (65), ledit cylindre intérieur (72) ayant l'espace pour recevoir le montage d'aiguille, le ressort de rappel (40), le piston escamotable vers l'intérieur capable de se casser (60) et le joint d'étanchéité (50) du piston.
